# EUROPEAN PATENT SPECIFICATION

(11) **EP 0 390 112 B1**
(45) Date of publication and mention of the grant of the patent: **28.01.1998**
(21) Application number: 90105920.4
(22) Date of filing: 28.03.1990
(51) Int. Cl.: C07D 473/00, C07D 487/04, C07H 19/167, A61K 31/52, A61K 31/70, A61K 31/505

(54) **Selective adenosine receptor agents**
Selektive Adenosinrezeptor-Liganden
Composés sélectifs pour le récepteur de l'adénosine

(30) Priority: 29.03.1989 US 329919
(43) Date of publication of application: 03.10.1990
(73) Proprietor: MERRELL PHARMACEUTICALS INC., Cincinnati, Ohio 45215-6300 (US)
(72) Inventor: Peet, Norton P., Cincinnati, Ohio 45241 (US); Lentz, Nelsen L., West Chester, Ohio 45069 (US); Sunder, Shyam, Indianapolis, Indiana (US)
(74) Representative: VOSSIUS & PARTNER

(56) References cited:
- EP-A- 0 181 129
- EP-A- 0 212 535
- EP-A- 0 287 907
- EP-A- 0 300 144
- EP-A- 0 300 145
- WO-A-90/09178
- DE-A- 1 795 828
- DE-A- 2 139 107
- GB-A- 953 897
- GB-A- 1 123 245
- GB-A- 2 077 726
- US-A- 3 862 189
- US-A- 4 514 405
- JOURNAL OF THE AMERICAN CHEMICAL SOCIETY, vol. 78, no. 15, 6th August 1956, pages 3693-3696, American Chemical Society; M.W. BULLOCK et al.: "Syntheses of 6-substituted purines"
- JOURNAL OF MEDICINAL CHEMISTRY, vol. 28, no. 11, November 1985, pages 1636-1643, American Chemical Society; S. KUSACHI et al.: "Dog coronary artery adenosine receptor: Structure of the N6-alkyl subregion"
- BIOCHEMICAL PHARMACOLOGY, vol. 35, no. 15, 1st August 1986, pages 2467-2481, Pergamon Press Ltd, Oxford, GB; J.W. DALY et al.: "Structure-activity relationships for N6-substituted adenosines at a brain A1-adenosine receptor with a comparison to an A2-adenosine receptor regulating coronary blood flow"
- GENERAL PHARMACOLOGY, vol. 18, no. 4, 1987, pages 405-416, Pergamon Journals Ltd, GB; R.A. BARRACO et al.: "The effects of parenteral injections of adenosine and its analogs on blood pressure and heart rate in the rat"
- JOURNAL OF MEDICINAL CHEMISTRY, vol. 18, no. 8, August 1975, pages 780-783, American Chemical Society; S.P. DUTTA et al.: "Synthesis and biological activities of some N6-(Nitro- and -aminobenzyl)adenosines"
- ARZNEIMITTEL FORSCHUNG, vol. 20, no. 11, November 1970, pages 1749-1751, Aulendorf i. Württ, DE; K. DIETMANN et al.: "Hemmung der induzierten Thrombocyten-Aggregation durch Adenosin und Adenosin-Derivate. II. Korrelation zwischen Hemmung der Aggregation und peripherer Vasodilatation"
- CHEMICAL ABSTRACTS, vol. 105, no. 23, 8th December 1986, page 72, abstract no. 203346b, Columbus, Ohio, US; D.M. PATON et al.: "Nature of the N6 region of the adenosine receptor in guinea pig ileum and rat vas deferens" & NAUNYN- SCHMIEDEBERG'S ARCH. PHARMACOL. 1986, 333(3), 313-22
- CHEMICAL ABSTRACTS, vol. 94, no. 13, 30th March 1981, page 271, abstract no. 98077u, Columbus, Ohio, US; E.A. RISLEY: "Biochemical characterization of putative central purinergic receptors by using 2-chloro(3H)-adenosine, a stable analog of adenosine" & PROC. NATL. ACAD. SCI. U.S.A. 1980, 77(11), 6892-6

## Description

The present invention relates to a group of compounds which are adenosine analogues and which act selectively at adenosine receptors.

The profound hypotensive, sedative, antispasmodic, and vasodilatory actions of adenosine were first recognized over 50 years ago. Subsequently, the number of biological roles proposed for adenosine have increased considerably. The adenosine receptors appear linked in many cells to adenylate cyclase. A variety of adenosine analogues have been introduced in recent years for the study of these receptor functions. Alkylxanthines, such as caffeine and theophylline, are the best known antagonists of adenosine receptors.

Adenosine perhaps represents a general regulatory substance, since no particular cell type or tissue appears uniquely responsible for its formation. In this regard, adenosine is unlike various endocrine hormones. Nor is there any evidence for storage and release of adenosine from nerve or other cells. Thus, adenosine is unlike various neurotransmitter substances.

Adenosine might be compared as a physiological regulator to the prostaglandins. In both cases the enzymes involved in the metabolic formation are ubiquitous and appear to be responsive to alterations in the physiological state of the cell. Receptors for adenosine, like those for prostaglandins, are proving to be very widespread. Finally, both prostaglandins and adenosine appear to be involved with the regulation of functions involving calcium ions. Prostaglandins, of course, derive from membrane precursors, while adenosine derives from cytosolic precursors.

Although adenosine can affect a variety of physiological functions, particular attention has been directed over the years toward actions which might lead to clinical applications. Preeminent has been the cardiovascular effects of adenosine which lead to vasodilation and hypotension but which also lead to cardiac depression. The antilipolytic, antithrombotic and antispasmodic actions of adenosine have also received some attention. Adenosine stimulates steroidogenesis in adrenal cells, again probably via activation of adenylate cyclase. Adenosine has inhibitory effects on neurotransmission and on spontaneous activity of central neurons. Finally, the bronchoconstrictor action of adenosine and its antagonism by xanthines represents an important area of research.

It has now been recognized that there are not one but at least two classes of extracellular receptors involved in the action of adenosine. One of these has a high affinity for adenosine and at least in some cells couples to adenylate cyclase in an inhibitory manner. These have been termed by some as the A-1 receptors. The other class of receptors has a lower affinity for adenosine and in many cell types couples to adenylate cyclase in a stimulatory manner. These have been termed the A-2 receptors.

Characterization of the adenosine receptors has now been possible with a variety of structural analogues. Adenosine analogues resistant to metabolism or uptake mechanisms have become available. These are particularly valuable, since their apparent potencies will be less affected by metabolic removal from the effector system. The adenosine analogues exhibit differing rank orders of potencies at A-1 and A-2 adenosine receptors, providing a simple method of categorizing a physiological response with respect to the nature of the adenosine receptor. The blockade of adenosine receptors (antagonism) provides another method of categorizing a response with respect to the involvement of adenosine receptors. It should be noted that the development of antagonists specific to A-1 or A-2 adenosine receptors would represent a major breakthrough in this research field and in the preparation of adenosine receptor selective pharmacological agents having specific physiological effects in animals.

In Journal of Am. Chem. Soc. 78 (1956) 3693-3696, EP-A-0 212 535, EP-A-0 181 129, GB-A-953 897, EP-A-0 287 907, US-A-4 514 405, DE-A-2 139 107, DE-A-1 795 828, GB-A-2 077 726, US-A-3 862 189, GB-A-1 123 245, Journal of Med. Chem. 28 (1985) 1636-1643, Biochemical Pharmacology 35 (1986) 2467-2481, General Pharmacology 18 (1987) 405-416, EP-A-0 300 144, EP-A-0 300 145, Chem. Abs. 94 (1981) Abs. no. 98077u, Chem. Abs. 105 (1986) Abs. no. 203346b, Journal of Med. Chem. 18 (1975) 780-783 and Arzneimittel Forschung 20 (1970) 1749-1751 various adenosine analogues are disclosed.

The present invention relates to compounds having the following general formulae: 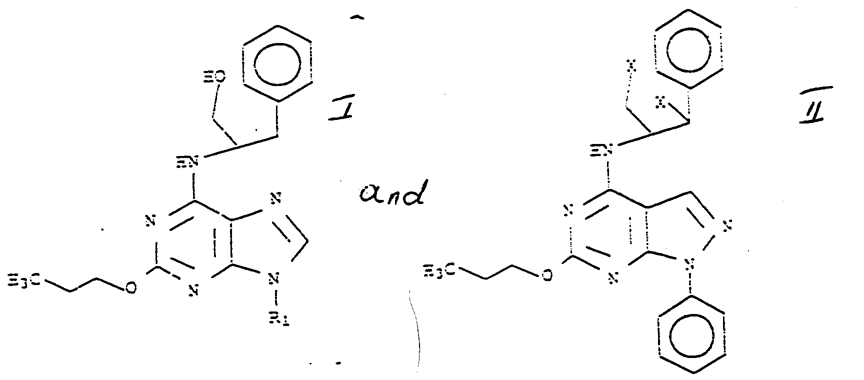 wherein R₁ is hydrogen, phenyl or β-*D*-ribofuranosyl; and each X is independently hydrogen or hydroxy,

Stereoisomerism is possible with the present compounds and the chemical structure as presented above is considered as encompassing all of the possible stereoisomers and racemic mixtures of such stereoisomers. More specifically, when X as shown in Formula II is other than hydrogen, chirality is exhibited about the respective carbon atom and optical isomerism is possible.

As examples of compounds of the present invention are the following:
1. (R)-N-(1-methyl-2-phenylethyl)-1-phenyl-6-propoxy-1H-pyrazolo[3,4-d]pyrimidin-4-amine
2. (S)-N-(1-methyl-2-phenylethyl)-1-phenyl-6-propoxy-1H-pyrazolo[3,4-d]pyrimidin-4-amine
3. (S)-β-[(1-phenyl-6-propoxy-1H-pyrazolo[3,4-d]pyrimidin-4-yl)amino]benzenepropanol
4. (R)-β-[(1-phenyl-6-propoxy-1H-pyrazolo[3,4-d]pyrimidin-4-yl)amino]benzenepropanol
5. (R)-β-[(6-propoxy-9-β-*D*-ribofuranosyl-9H-purin-6-yl)amino]benzenepropanol
6. (S)-β-[(6-propoxy-9-β-*D*-ribofuranosyl-9H-purin-6-yl)amino]benzenepropanol

In general, the compounds of the present invention are formed by reacting, under appropriate conditions, a compound of the general structure: 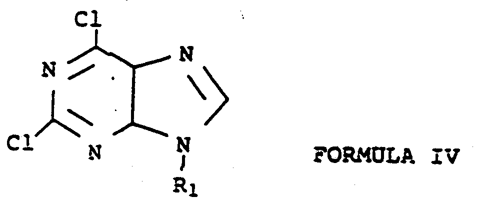 wherein R₁ is hydrogen or phenyl with propanol by contacting the compound according to FORMULA IV with propanol in ethanol in the presence of triethylamine for a period of from 1 to 20 hours at a temperature of from 25°C to 140°C; to obtain the intermediate product according to FORMULA V therefrom: 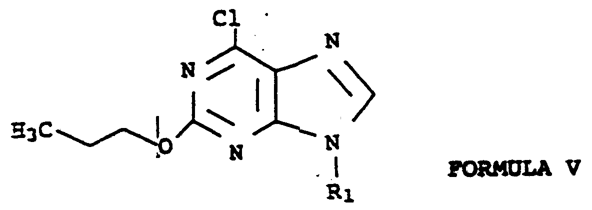 and by then reacting a compound according to FORMULA V with a compound according to FORMULA III for a period of from 1 to 20 hours at a temperature of from 25°C to 140°C; to obtain the desired product according to FORMULA I therefrom

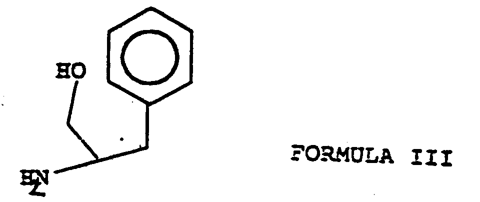

Compounds of Formula II may be prepared by reacting a compound according to FORMULA IV' 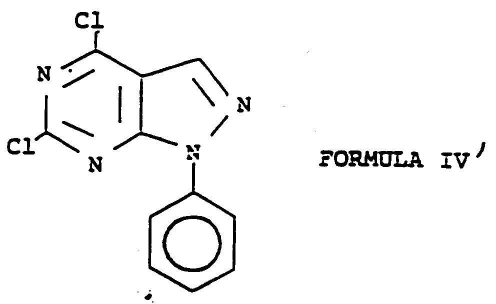 with propanol contacting the compound according to FORMULA IV' with propanol in ethanol in the presence of triethylamine for a period of from 1 to 20 hours at a temperature of from 25°C to 140°C; to obtain the intermediate product according to FORMULA V' therefrom: 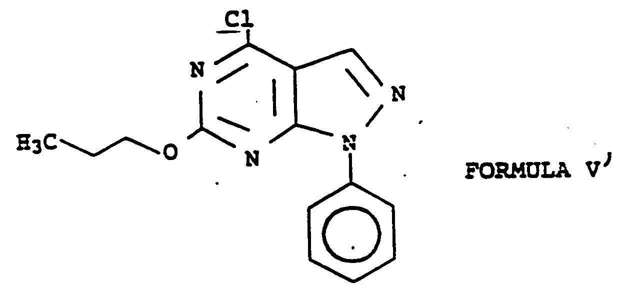 and by then reacting a compound according to FORMULA V' with a compound according to FORMULA III' for a period of from 1 to 20 hours at a temperature of from 25°C to 140°C; to obtain the desired product according to FORMULA II therefrom. 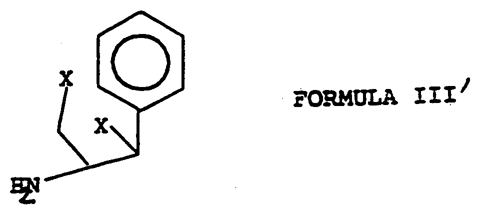 wherein each X is independently either hydrogen or hydroxy.

### Therapeutic Utility Of Selective Adenosine Receptor Agents

The table below shows in more detail the potential therapeutic utility of selective adenosine receptor agents in accordance with the present invention:

| Area | Effect | Receptor Correlate |
|---|---|---|
| Cardiovascular | cardiotonic | A-1 antagonism |
| Cardiovascular | control tachycardia | A-1 agonism |
| Cardiovascular | increase coronary blood flow | A-2 agonism |
| Cardiovascular | vasodilation | A-2 (atypical) agonism |
| Pulmonary | bronchodilation | A-1 antagonism |
| Pulmonary | mediation of autocoid release from mast cells, basophils | novel adenosine receptor interaction on cell surface |
| Pulmonary | stimulate respiration; treat paradoxical ventilatory response (infants) | Ado antagonism |
| Renal | inhibit renin release | A-1 agonism |
| Central Nervous System | aid in opiate withdrawal | Ado agonism |
| Central Nervous System | analgesic | A-1 agonism |
| Central Nervous System | anticonvulsant | A-1 agonism |
| Central Nervous System | antidepressant | A-1 agonism |
| Central Nervous System | antipyschotic | Ado agonism |
| Central Nervous System | anxiolytic | agonism |
| Central Nervous System | inhibition of self-mutilation behavior (Lesch-Nyhan syndrome) | Ado agonism |
| Central Nervous System | sedative | A-2 agonism |

In the cardiovascular, pulmonary and renal system targets, designed compounds which are identified by receptor binding studies can be evaluated in functional *in vivo* tests which are directly indicative of the human physiological response. A good description of the pharmacology and functional significance of purine receptors is presented by M. Williams in Ann. Rev. Pharmacol. Toxicol., 27, 31 (1987), which is incorporated herein by reference. In a section entitled "Therapeutic Targeting of Adenosine Receptor Modulators" it is stated that "adenosine agonists may be effective as antihypertensive agents, in the treatment of opiate withdrawal, as modulators of immune competence and renin release, as antipsychotics and as hypnotics. Conversely, antagonists may be useful as central stimulants, inotropics, cardiotonics, antistress agents, antiasthmatics, and in the treatment of respiratory disorders." The smorgasbord of activities displayed by adenosine receptor agents underscores their great potential utility for therapy and the need for central agents.

Adenosine exerts its various biological effects via action on cell-surface receptors. These adenosine receptors are of two types: A-1 and A-2. The A-1 receptors are operationally defined as those receptors at which several N6-substituted adenosine analogs such as R-phenylisopropyladenosine (R-PIA) and cycloadenosine (CHA) are more potent than 2-chloroadenosine and N-5'-ethylcarboxamidoadenosine (NECA). At A-2 receptors the order of potency is instead NECA>2-chloroadenosine>R-PIA>CHA.

As illustrated in the table above, adenosine receptors govern a variety of physiological functions. The two major classes of adenosine receptors have already been defined. These are the A-1 adenosine receptor, which is inhibitory of adenylate cyclase, and the A-2 adenosine receptor, which is stimulatory to adenylate cyclase. The A-1 receptor has a higher affinity for adenosine and adenosine analogs than the A-2 receptor. The physiological effects of adenosine and adenosine analogs are complicated by the fact that non-selective adenosine receptor agents first bind the rather ubiquitous low-affinity A-2 receptors, then as the dose is increased, the high-affinity A-2 receptors are bound, and finally, at much higher doses, the very high-affinity A-1 adenosine receptors are bound. (See J. W. Daly, et al., *Subclasses of Adenosine Receptors in the Central Nervous System: Interaction with Caffeine and Related Methylxanthines,* Cellular and Molecular Neurobiology, 3,(1), 69-80 (1983), incorporated herein by reference.)

In general, the physiological effects of adenosine are mediated by either the stimulation or the inhibition of adenylate cyclase. Activation of adenylate cyclase increases the intracellular concentration of cyclic AMP, which, in general, is recognized as an intracellular second messenger. The effects of adenosine analogs can therefore be measured by either the ability to increase or the ability to antagonize the increase in the cyclic AMP in cultured cell lines. Two important cell lines in this regard are VA 13 (WI-38 VA 13 2RA), SV-40 transformed WI 38 human fetal lung fibroblasts, which are known to carry the A-2 subtype of adenosine receptor, and fat cells, which are known to carry the A-1 subtype of adenosine receptor. (See R.F. Bruns, *Adenosine Antagonism by Purines, Pteridines and Benzopteridines in Human Fibroblasts,* Chemical Pharmacology, 30, 325-33, (1981), incorporated herein by reference.)

It is well known from *in vitro* studies that the carboxylic acid congener of 8-phenyl-1,3-dipropyl-xanthine (XCC) is adenosine receptor nonselective, with a Ki at the A-1 receptors in brain membranes of 58± 3nM and a Ki at the A-2 receptors of the brain slice assay of 34± 13nM. The amino congener of 8-phenyl-1,3-dipropyl-xanthine (XAC), on the other hand, has a 40-fold higher affinity for A-1 adenosine receptors, with a Ki of 1.2± 0.5nM, as compared with a Ki at the A-2 receptors of the brain slice assay of 49± 17nM. In addition, XAC is much more potent in antagonizing the effects of adenosine analogs on heart rate than on blood pressure. Since it is generally known that the adenosine analog-induced effects on the heart seem to be mediated via A-1 receptors and those on blood pressure via A-2 receptors, the selectivity of XAC under *in vivo* conditions suggests that adenosine receptor activity *in vitro* correlates with adenosine receptor activity *in vivo* and that specific physiological effects can be distinguished as a result of this selectivity. (See B.B. Fredholm, K.A. Jacobsen, B. Jonzon, K.L. Kirk, Y.O. Li, and J.W. Daly, *Evidence That a Novel 8-Phenyl-Substituted Xanthine Derivative is a Cardioselective Adenosine Receptor Antagonist In Vivo*, Journal of Cardiovascular Pharmacology, 9, 396-400, (1987), incorporated herein by reference and also K.A. Jacobsen, K.L. Kirk, J.W. Daly, B. Jonzon, Y.O. Li, and B.B. Fredholm, *Novel 8-Phenyl-Substituted Xanthine Derivative Is Selective Antagonist At Adenosine Receptors In Vivo,* Acta Physiol. Scand., 341-42, (1985), incorporated herein by reference.)

It is also known that adenosine produces a marked decrease in blood pressure. This blood pressure reduction is probably dependent upon an A-2 receptor-mediated decrease in peripheral resistance. Adenosine analogs are also able to decrease heart rate. This effect is probably mediated via adenosine receptors of the A-1 subtype.

Thus, it is readily apparent that the pharmacological administration of the adenosine receptor selective adenosine analogs disclosed herein will result in selective binding to either the A-2 or the A-1 receptor, which will, in turn, selectively result in either a decrease in blood pressure or a decrease in heart rate, for example, thereby decoupling these physiological effects *in vivo*. The selection of such adenosine receptor selective agents can be determined by the methods described in further detail below.

### Test For Affinity For Brain Adenosine A-2 Receptors

The test described below was used to determine the potency of test compounds to compete with the ligand [3H]5′-N-ethyl-carboxamidoadenosine (NECA) for the adenosine A-2 receptors prepared from animal brain membranes. (See also R.R. Bruns, G.H. Lu, and T.A. Pugsley, *Characterization of the A-2 Adenosine Receptor Labeled by [3H]NECA in Rat Striatal Membranes,* Mol. Pharmacol., 29, 331-346, (1986), incorporated herein by reference.) Young male rats (C-D strain), obtained from Charles River, are killed by decapitation and the brain was removed. Membranes for ligand binding are isolated from rat brain striatum. The tissue is homogenized in 20 vol ice-cold 50 mM Tris-HCl buffer (pH 7.7) using a polytron (setting 6 for 20 seconds). The homogenate is centrifuged at 50,000 x g for 10 minutes at 4°C. The pellet is again homogenized in a polytron in 20 vol of buffer, and centrifuged as before. The pellet is finally resuspended in 40 vol of 50mM Tris-HCl (pH 7.7) per gram of original wet weight of tissue.

Incubation tubes, in triplicate, receive 100 µl of [3H]NECA (94 nM in the assay), 100 µl of 1 µM cyclohexyladenosine (CHA), 100 µl of 100 mM MgCl₂, 100 µl of 1 IU/ml adenosine deaminase, 100 µl of test compounds at various concentrations over the range of 10⁻¹⁰ M to 10⁻⁴ M diluted with assay buffer (50 mM Tris-HCl, pH 7.7) and 0.2 µl of membrane suspension (5 mg wet weight), in a final volume of 1 ml of 50 mM Tris-HCl, pH 7.7. Incubations are carried out at 25°C for 60 minutes. Each tube is filtered through GF/B glass fiber filters using a vacuum. The filters are rinsed two times with 5 ml of the ice-cold buffer. The membranes on the filters are transferred to scintillation vials to which 8 ml of Omnifluor with 5% Protosol is added. The filters are counted by liquid scintillation spectrometry.

Specific binding of [3H]NECA is measured as the excess over blanks run in the presence of 100 µM 2-chloroadenosine. Total membrane-bound radioactivity is about 2.5% of that added to the test tubes. Since this condition limits total binding to less than 10% of the radioactivity, the concentration of free ligand does not change appreciably during the binding assay. Specific binding to membranes is about 50% of the total bound. Protein content of the membrane suspension is determined by the method of O.H. Lowry, N.J. Rosebrough, A.L. Farr and R.J. Randall, *Protein Measurements With Folin Phenol Reagent,* J. Biol. Chem., 193, 265-275 (1951), (incorporated herein by reference).

Displacement of [3H]NECA binding of 15% or more by a test compound is indicative of affinity for the adenosine A-2 site. The molar concentration of a compound which causes 50% inhibition of the binding of ligand is the IC₅₀. A value in the range of 100-1000 nM would indicate a highly potent compound.

### Test For Affinity For Brain Adenosine A-1 Receptor Binding Sites

The test described below is used to determine the potency of test compounds to compete with the ligand [3H]cycloadenosine for the Adenosine A-1 receptor prepared from rat brain membranes. Male Sprague-Dawley rats are sacrificed by decapitation and the membranes are isolated from whole animal brains. (See R. Goodman, M. Cooper, M. Gavish, and S. Synder, *Guanine Nucleotide and Cation Regulation of the Binding of [3H] Diethylphenylxanthine to Adenosine A-1 Receptors in Brain Membrane,* Molecular Pharmacology, 21, 329-335, (1982), incorporated herein by reference.)

Membranes are homogenized (using polytron setting 7 for 10 seconds) in 25 volumes of ice-cold 50 mM Tris-HCl buffer, pH 7.7. The homogenate is centrifuged at 19,000 rpm for 10 minutes at 4°C. The pellet is washed by resuspending in 25 volumes of buffer with 2 IU of adenosine deaminase per ml and incubated 30 minutes at 37°C. The homogenate is centrifuged again. The final pellet is resuspended in 25 volumes of ice-cold buffer.

The incubation tubes, in triplicate, receive 100 µl of [3H]cyclohexyladenosine, 0.8 nM in the assay, 200 µl of test compounds at various concentrations over the range of 10⁻¹⁰ M to 10⁻⁶ M diluted with 50 nM Tris-HCl buffer (pH 7.7), 0.2 ml of membrane suspension (8 mg wet weight) and in a final volume of 2 ml with Tris buffer. Incubations are carried out at 25°C for 2 hours and each one is terminated within 10 seconds by filtration through a GF/B glass fiber filter using a vacuum. The membranes on the filters are transferred to scintillation vials. The filters are counted by liquid scintillation spectometry in 8 ml of Omniflour containing 5% Protosol.

Specific binding of [3H]cycloadenosine is measured as the excess over blanks taken in the presence of 10⁻⁵ M 2-chloroadenosine. Total membrane-bound radioactivity is about 5% of that added to the test tubes. Specific binding to membranes is about 90% of the total bound. Protein content of the membrane suspension is determined by the method of Lowry, et al. Id., 265.

Displacement of [3H]cyclohexyladenosine binding of 15% or more by a test compound is indicative of affinity for the adenosine binding site.

### Adenosine Receptor Binding Affinity Values Obtained Using The Above Described Test Procedures

The following is a table showing the adenosine receptor binding affinities for several compounds (refer to compound examples on page 4 for cross reference to compound names) within the scope of the present invention:

| Compound | A-1 Receptor Ki | A-2 Receptor Ki | A-2Ki/A-1 Ki |
|---|---|---|---|
| 1. | 2.01 x 10⁻⁶ | 3.63 x 10⁻⁶ | 1.80 |
| 2. | 1.13 x 10⁻⁵ | >6.99 x 10⁻⁶ | -- |
| 3. | 1.74 x 10⁻⁶ | 2.90 x 10⁻⁶ | 1.67 |
| 4. | 3.21 x 10⁻⁷ | 3.77 x 10⁻⁷ | 1.17 |
| 5. | 3.70 x 10⁻⁶ | 1.72 x 10⁻⁵ | 4.65 |
| 6. | 4.40 x 10⁻⁸ | 1.90 x 10⁻⁶ | 43.18 |

The nucleotide guanosine triphosphate (GTP) has been shown to differentially affect the binding of agonists and antagonists to a variety of neurotransmitter receptors. In general, guanine nucleotides lower the affinity of agonists for receptors without a concomitant decrease in antagonist affinity. Accordingly, GTP has been shown to decrease the potency of agonists but not antagonists as inhibitors of the binding of the adenosine antagonist [3H]3-diethyl-8-phenyl-xanthine. In general, GTP greatly reduces the potency of purine agonists, but not antagonists as inhibitors of [3H]-phenylisopropyl adenosine binding and is, therefore, an effective agent for distinguishing between agonists and antagonists. (See L.P. Davies, S.C. Chow, J.H. Skerritt, D.J. Brown and G.A.R. Johnston, *Pyrazolo [3,4-d]Pyrimidines as Adenosine Antagonists*, Life Sciences, 34, 2117-28, (1984), incorporated herein by reference.) It is understood, in general, that adenosine analogs act as agonists if β-*D*-ribofuranosyl is present in the molecule at the R₁ position and as an antagonist if R₁ is hydrogen or phenyl.

### Pharmaceutical Preparations of the Adenosine Receptor Selection Adenosine Analogs

The exact amount of the compound or compounds to be employed, i.e., the amount of the subject compound or compounds sufficient to provide the desired effect, depends on various factors such as the compound employed; type of administration; the size, age and species of animal; the route, time and frequency of administration; and, the physiological effect desired. In particular cases, the amount to be administered can be ascertained by conventional range finding techniques.

The compounds are preferably administered in the form of a composition comprising the compound in admixture with a pharmaceutically acceptable carrier, i.e., a carrier which is chemically inert to the active compound and which has no detrimental side effects or toxicity under the conditions of use. Such compositions can contain from about 0.1 µg or less to 500 mg of the active compound per ml of carrier to about 99% by weight of the active compound in combination with a pharmaceutically-acceptable carrier.

The compositions can be in solid forms, such as tablets, capsules, granulations, feed mixes, feed supplements and concentrates, powders, granules or the like; as well as liquid forms such as sterile injectable suspensions, orally administered suspensions or solutions. The pharmaceutically acceptable carriers can include excipients such as surface active dispersing agents, suspending agents, tableting binders, lubricants, flavors and colorants. Suitable excipients are disclosed, for example, in texts such as Reminaton's Pharmaceutical Manufacturing, 13 Ed., Mack Publishing Co., Easton, Pennsylvania (1965).

The following examples are presented to illustrate the present invention.

### EXAMPLE 1

To a solution of 2.5 g of 2,6-dichloropurine dissolved in 50 ml ethanol was added 2.0 g (S)-(-)-2-amino-3-phenyl-1-propanol, and 1.83 ml Et₃N with stirring at room temperature for 2 hours. The mixture was then heated to reflux for 20 hours. The solvent was removed under vacuum and the residue purified by flash chromatography (5-10% MeOH/CHCl₃) to yield 3.68 g of a yellow solid, S-β-[(2-chloro-1H-purin-6-yl)amino]benzenepropanol (m.p. 117-123°C).

This was followed by suspension of 1.42 g of the above product in 30 ml CHCl₃ and treatment with 1.30 g triphenylmethylchloride and 0.65 ml Et₃N. After 3 hours the reaction was diluted with 200 ml CHCl₃, remixed with 200 ml saturated NaHCO₃ and 200 ml saturated NaCl, dried over MgSO₄, filtered and concentrated to yield 3.3 g of a yellow solid. This was flash chromatographed (5% MeOH/CHCl₃) to yield 2.16 g of a foam product, S-β-[(9-triphenylmethyl-2-chloro-1H-purin-6-yl)amino]benzenepropanol.

To a solution of 330 mg sodium dissolved in 100 ml n-propanol was added 2.15 g of S-β-[(9-triphenylmethyl-2-chloro-1H-purin-6-yl)amino]benzenepropanol and the reaction was heated to reflux for 6 hours. It was then cooled to room temperature, poured into 300 ml H₂O and extracted with CHCl₃ (3 times 200 ml). The combined organic extracts were washed with 300 ml saturated NaCl, dried over Na₂SO₄, filtered and concentrated to yield 2.16 g of a foam product, S-β-[(2-propoxy-9-triphenylmethyl-1H-purin-6-yl)amino]benzenepropanol.

Subsequently, 2.14 g of S-β-[(2-propoxy-9-triphenylmethyl-1H-purin-6-yl)amino]benzenepropanol was dissolved in 50 ml CH₂Cl₂ followed by addition of p-toluenesulfonic acid (0.71g). After stirring 24 hours the solvent was removed under vacuum and the residue was purified by flash chromatography (5-10% MeOH/CHCl₃) to yield 0.81 g of a white solid, (S)-β-[(2-propoxy-1H-purin-6-yl)amino]benzenepropanol (m.p. 229-231°C).

### EXAMPLE 2

1.94 g of D-amphetamine sulfate was made basic with 10% KOH. The aqueous solution was extracted with ether. The organic layer was dried over MgSO₄, filtered and concentrated to yield a clear oil. This was diluted with 3 ml of ethanol and added to a stirred solution of 466 g of 1-phenyl-4,6-dichloropyrazolo(3,4-d]pyrimidine in 7 ml ethanol. After 48 hours the solvent was removed under vacuum and the crude material was purified by radial chromatography (20%-40% ethyl alcohol/hexane, 2mm plate) to yield 640 mg of (S)-N-(1-methyl-2-phenylethyl)-1-phenyl-6-chloro-1H-pyrazolo[3,4-d]pyrimidin-4-amine (100%).

Next, 324 mg sodium was reacted with 10 ml of n-propanol. To this, 640 mg of (S)-N-(1-methyl-2-phenylethyl)-1-phenyl-6-chloro-1H-pyrazolo[3,4-d]pyrimidin-4-amine in 5 ml of n-propanol was added with stirring and the reaction was heated to 90°C for 2 hours. It was then cooled, diluted with 200 ml saturated NaCl and extracted with 200 ml CHCl₃. The organic layer was dried over MgSO₄, filtered and concentrated to yield an oil which was purified by radial chromatography (30-50% Et₂O/hexane) to yield after recrystallization from 30% Et₂O/hexane 382 mg of product (m.p. 134-136°C). Proton NMR indicated ether was still present. The compound was then oven dried under vacuum for 6 hours to yield 318 mg of (S)-N-(1-methyl-2-phenylethyl)-1-phenyl-6-propoxy-1H-pyrazolo[3,4-d]pyrimidin-4-amine (m.p. 134-136°C).

### EXAMPLE 3

1.94 g of L-amphetamine sulfate was dissolved in H₂O, made basic and extracted with ether. The ether layer was concentrated under vacuum, the oil was taken up in 3 ml ethanol and added to a stirred suspension of 465 mg 1-phenyl-4,6-dichloropyrazolo[3,4-d]pyrimidine in 7 ml ethanol. After 24 hours the solvent was removed under vacuum and the crude oil was purified by radial chromatography (40-60% Et₂O/hexane, 2 mm plate) to yield 531 mg of (R)-N-(1-methyl-2-phenylethyl)-1-phenyl-6-chloro-1H-pyrazolo[3,4-d]pyrimidin-4-amine (83%).

Next, 268 mg sodium was reacted with 10 ml of n-propanol. 531 mg of (R)-N-(1-methyl-2-phenylethyl)-1-phenyl-6-chloro-1H-pyrazolo[3,4-d]pyrimidin-4-amine in 5 ml n-propanol was added to the stirred solution under nitrogen. The reaction was heated (oil bath 90°C) for 2 hours. It was then cooled, diluted with 100 ml saturated NaCl and the aqueous solution was extracted with 200 ml CHCl₃. The organic layer was then dried over MgSO₄, filtered and concentrated to yield an oil which was purified by radial chromatography (30-50% Et₂O/hexane, 2mm plate) to yield after recrystallisation from 30% Et₂O/hexane 381.4 mg of a white solid (m.p. 135-137°C). Proton NMR indicated ether was still present. Thus the compound was oven dried under vacuum (setting at 3) for 6 hours to yield 326 mg of final product, (R)-N-(1-methyl-2-phenylethyl)-1-phenyl-6-propoxy-1H-pyrazolo[3,4-d]pyrimidin-4-amine (m.p. 135-137°C).

### EXAMPLE 4

1 g of 1-phenyl-4,6-dichlorpyrazolo[3,4-d]pyrimidine was suspended in 25 ml ethanol. 1.71 g of 1R,2S-norephedrine was added with stirring. After 24 hours the solvent was removed under vacuum and the crude oil was purified by radial chromatography (40-50-60-70% Et₂O/hexane, 4mm plate) to yield 1.17 g of a white solid, [S-(R*,S*)]-α-[1-[(1-phenyl-6-chloro-1H-pyrazolo[3,4-d]pyrimidin-4-yl)amino]ethyl]benzenemethanol (m.p. 164-165°C, 82% yield).

Next, 194 mg sodium was reacted with 10 ml of n-propanol. 400 mg of [S-(R*,S*)]-α-[1-[(1-phenyl-6-chloro-1H-pyrazolo[3,4-d]pyrimidin-4-yl)amino]ethyl]benzenemethanol in 5 ml of n-propanol was added to the stirred solution under nitrogen. The reaction was heated to 90°C for 2 hours. It was then diluted with 100 ml saturated NaCl, extracted with 200 ml CHCl₃, filtered and concentrated under vacuum to yield an oil which was purified by radial chromatography (5-10-20% isopropyl alcohol/hexane, 4mm plate) to yield 423 mg of an oil. Recrystallization from 20% Et₂O/hexane and vacuum oven drying at 70°C for 24 hours yield 122 mg of [S-(R*,S*)]-α-[1-[(1-phenyl-6-propoxy-1H-pyrazolo[3,4-d]pyrimidin-4-yl)amino]ethyl]benzenemethanol (m.p. 136-138°C).

### EXAMPLE 5

First, 2.5 g of 1-phenyl-4,6-dichloropyrazolo[3,4-d]pyrimidine was suspended in 60 ml ethanol, then 4.28 g (S)-(-)-2 amino-3-phenyl-1-propanol was added and the reaction was allowed to stir for 24 hours. The solvent was then removed under vacuum and the crude oil was purified by flash chromatography (10-15-20% isopropyl alcohol/hexane) to yield 3.5 g of product (S)-β-[(1-phenyl-6-chloro-1H-pyrazolo[3,4-d]pyrimidin-4-yl)amino]benzenepropanol (97%).

Next, 314 mg sodium was reacted with 15 ml of n-propanol. 650 mg of (S)-β-[(1-phenyl-6-chloro-1H-pyrazolo[3,4-d]pyrimidin-4-yl)amino]benzenepropanol dissolved in 10 ml of n-propanol was added to the reaction with stirring under nitrogen. The reaction was then heated to 90°C for two hours. After cooling it was poured into 100 ml saturated NaCl and extracted with 200 ml CHCl₃. The organic layer was dried over MgSO₄, filtered and concentrated to yield an oil which was purified by radial chromatography (10-20% isopropyl alcohol/hexane) to yield after recrystallization from 30% isopropyl alcohol/hexane and oven drying under vacuum at 60°C for 72 hours, 319 mg of (S)-β-[(1-phenyl-6-propoxy-1H-pyrazolo[3,4-d]pyrimidin-4-yl)amino]benzenepropanol (46%, m.p. 155-157°C).

### EXAMPLE 6

First, 2.81 g of 1-phenyl-4,6-dichloropyrazolo[3,4-d]pyrimidine was suspended in 60 ml of ethanol, then 3.2 g of (R)-(+)-2-amino-3-phenyl-1-propanol was added with stirring. After 48 hours the solvent was removed under vacuum and the oil was flash chromatographed (2-5-7% MeOH/CHCl₃) to yield 3.80 g of (R)-β-[(1-phenyl-6-chloro-1H-pyrazolo[3,4-d]pyrimidin-4-yl)amino]benzenepropanol (95%).

Next, 380 mg of sodium was reacted with 10 ml of n-propanol. 773 mg of (R)-β-[(1-phenyl-6-chloro-1H-pyrazolo[3,4-d]pyrimidin-4-yl)amino]benzenepropanol in 5 ml of n-propanol was added with stirring. The reaction was heated to 90°C in an oil bath for 2.5 hours, then the solvent was removed under vacuum, the residue taken up in 200 ml CHCl₃. The organic layer was washed with saturated NaCl, dried over MgSO₄, filtered and concentrated to an oil which was purified by radial chromatography (10-20-30% isopropyl alcohol/hexane, 4mm plate) to yield after recrystallization from 30% isopropyl alcohol/hexane 217 mg of a white solid (m.p. 158-159°C). Proton NMR indicated n-propanol was still present, so this product was oven dried under vacuum for (setting at 3) to yield 161 mg of final product, (R)-β-[(1-phenyl-6-propoxy-1H-pyrazolo[3,4-d]pyrimidin-4-yl)amino]benzenepropanol (m.p. 157-158°C).

### EXAMPLE 7

First, 5 g of 2,6-dichloropurine and 8.4 g of ribose tetraacetate were combined and heated to 155°C with stirring to produce a heterogenous suspension. A drop of concentrated H₂SO₄ was added and the reaction was allowed to stir at 155°C until it became clear. The reaction was cooled and the HOAc was removed under vacuum. 30 ml of ethanol was added and trituration followed by filtration yielded 3.7 g of product. This was recrystallized from 175 ml ethanol to yield 2.31 g of 2,6-dichloro-9-(2,3,5-tri-*O*-acetyl-β-*D*-ribofuranosyl)-9H-purine with melting point of 154-156°C as long flat needles.

2.0 g of 2,6-dichloro-9-(2,3,5-tri-*O*-acetyl-β-*D*-ribofuranosyl)-9H-purine was combined with 0.67 g of S-(-)-2-amino-3-phenyl-1-propanol, 0.67 ml triethylamine and heated to reflux for 16 hours. The solvent was removed and the residue was purified by flash chromatography (5-10% methanol/trichloromethane) to yield 0.90 g of a foam. Less pure fractions were rechromatographed as above to provide a total of 1.81 g of (S)-β-[(9-(2,3,5-tri-*O*-acetyl-β-*D-*ribofuranosyl)-2-chloro-1H-purin-6-yl)amino]benzenepropanol.

Next, 0.59 g of sodium was reacted with 60 ml of n-propanol. The n-propoxide was then added to a stirring solution of 1.8 g of (S)-β-[(9-(2,3,5-tri-*O*-acetyl-β-*D*-ribofuranosyl)-2-chloro-1H-purin-6-yl)amino]benzenepropanol in 60 ml n-propanol and heated to reflux. After 16 hours, 3 ml water was added followed by MgSO₄. The reaction was filtered and concentrated under vacuum. The residue was purified by flash chromatography (20-30% methanol/trichloromethane) to yield 840 mg of product. This was recrystallized from about 20% isopropyl alcohol/hexane and dried under high vacuum for 7 days to yield 197 mg of (S)-β-[(2-propoxy-9-(β-*D*-ribofuranosyl)-1H-purin-6-yl)amino]benzenepropanol as a white solid (m.p. 102-104°C).

### EXAMPLE 8

First, 1.9 g of 2,6-dichloropurine and 3.2 g protected ribose (β-*D*-ribofuranose-1,2,3,5-tetraacetate) were combined and placed in an oil bath at 155°C. After 2 minutes of stirring a capillary drop of concentrated H₂SO₄ was added and the reaction became homogenous. After stirring an additional 10 minutes the reaction was cooled, the HOAc was removed under high vacuum and heat, and 15 ml of absolute ethanol added. The residue was dissolved with heat and the solution was placed in a freezer at -21°C. The white precipitate was collected and recrystallized from 100 ml absolute ethanol to yield after drying under vacuum at 80°C for 4 hours 2.16 g of 2,6-dichloro-9-(2,3,5-tri-*O*-acetyl-β-*D*-ribofuranosyl)-9H-purine having a melting point of 154-157°C.

21 g of 2,6-dichloro-9-(2,3,5-tri-*O*-acetyl-β-*D*-ribofuranosyl)-9H-purine was combined with 0.7 g of R-(+)-2-amino-3-phenyl-1-propanol, 0.7 ml triethylamine and 75 ml absolute ethanol and heated to reflux for 4 hours. The solvent was removed under vacuum and the residue purified by flash chromatography (5-10% methanol/trichloromethane) to yield 2.27 g of (R)-β-[(9-(2,3,5-tri-*O*-acetyl-β-*D*-ribofuranosyl)-2-chloro-1H-purin-6-yl)amino]benzenepropanol.

Next, 0.72 g of sodium was reacted in 150 ml n-propanol. This solution was added to 2.2 g of (R)-β-[(9-(2,3,5-tri-*O*-acetyl-β-*D*-ribofuranosyl)-2-chloro-1H-purin-6-yl)amino]benzenepropanol with stirring. The reaction was heated to reflux for 8 hours. After cooling the reaction was filtered and concentrated under vacuum. The residue was purified by flash chromatography (20-30% methanol/trichloromethane) to yield 930 mg of a foam product. This was recrystallized from about 10% isopropyl alcohol/hexane to yield after drying under vacuum at 80°C for 24 hours 590 mg of (R)-β-[(2-propoxy-9-(β-*D*-ribofuranosyl)-1H-purin-6-yl)amino]benzenepropanol as a white solid. (m.p. 149-152°C).

## Claims

1. A compound of the formula: 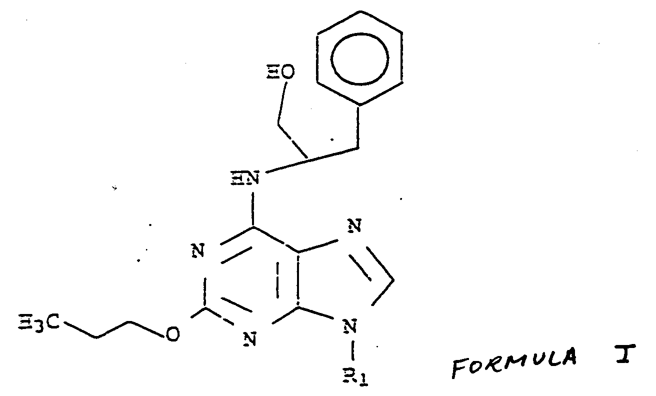 wherein R₁ is hydrogen or phenyl

2. A compound according to claim 1 which is β-[(2-propoxy-1H-purin-6-yl)amino]benzenepropanol.

3. A compound of the formula: 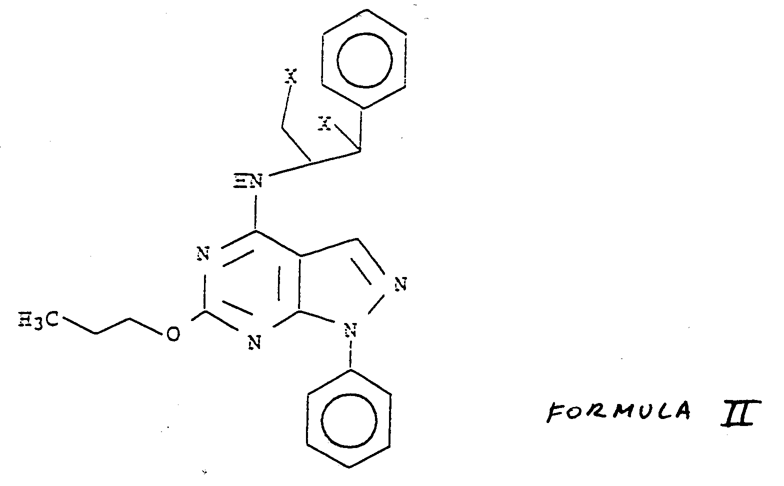 wherein each X is independently either hydrogen or hydroxy.

4. A compound according to claim 3 which is (R)-N-(1-methyl-2-phenylethyl)-1-phenyl-6-propoxy-1H-pyrazolo[3,4-d]pyrimidin-4-amine, (S)-N-(1-methyl-2-phenylethyl)-1-phenyl-6-propoxy-1H-pyrazolo[3,4-d]pyrimidin-4-amine, [S-(R*,-5*)]-α-[1-[(1-phenyl-6-propoxy-1H-pyrazolo[3,4-d]pyrimidin-4-yl)amino]ethyl]benzeneethanol, [R-(S*,-R*)]-α-[1-[(1-phenyl-6-propoxy-1H-pyrazolo[3,4-d]pyrimidin-4-yl)amino]ethyl]benzeneethanol, (S)-β-[(1-phenyl-6-propoxy-1H-pyrazolo[3,4-d]pyrimidin-4-yl)amino]benzenepropanol, or (R)-β-[(1-phenyl-6-propoxy-1H-pyrazolo[3,4-d]pyrimidin-4-yl)amino] benzenepropanol.

5. A compound according to any one of claims 1 to 4 for use as a therapeutic agent.

6. Pharmaceutical composition comprising a compound in accordance with any one of claims 1 to 4 and a physiologically acceptable carrier.

7. A process for the preparation of a pharmaceutical composition comprising combining a compound as defined in claims 1-4 with a pharmaceutically acceptable carrier.

8. A process for preparing a compound as defined in Claims 1 or 2 comprising:
reacting a compound according to FORMULA IV: 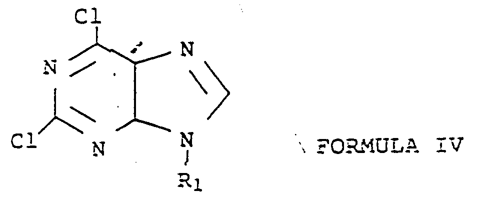 wherein R₁ is hydrogen or phenyl with propanol by contacting the compound according to FORMULA IV with propanol in ethanol in the presence of triethylamine for a period of from 1 to 20 hours at a temperature of from 25°C to 140°C; to obtain the intermediate product according to FORMULA V therefrom: 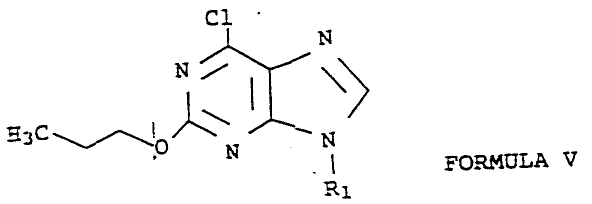 and by then reacting a compound according to FORMULA V with a compound according to FORMULA III for a period of from 1 to 20 hours at a temperature of from 25°C to 140°C; to obtain the desired product according to FORMULA I therefrom

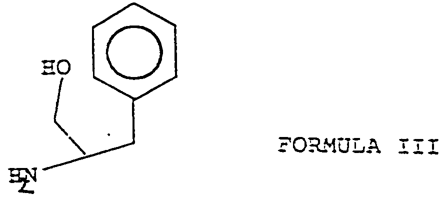

9. A process for preparing a compound according to Claims 3 or 4 comprising reacting a compound according to FORMULA IV' 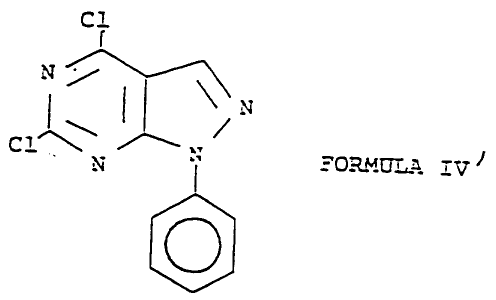 with propanol by contacting the compound according to FORMULA IV' with propanol in ethanol in the presence of triethylamine for a period of from 1 to 20 hours at a temperature of from 25°C to 140°C; to obtain the intermediate product according to FORMULA V' therefrom: 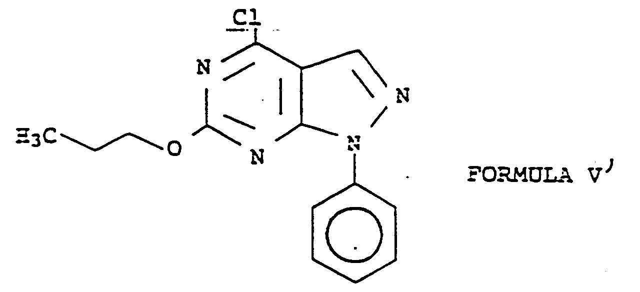 and by then reacting a compound according to FORMULA V' with a compound according to FORMULA III' for a period of from 1 to 20 hours at a temperature of from 25°C to 140°C; to obtain the desired product according to FORMULA II therefrom. 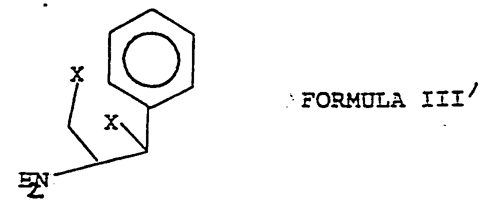 wherein each X is independently either hydrogen or hydroxy.

## Patentansprüche

1. Verbindung der Formel, 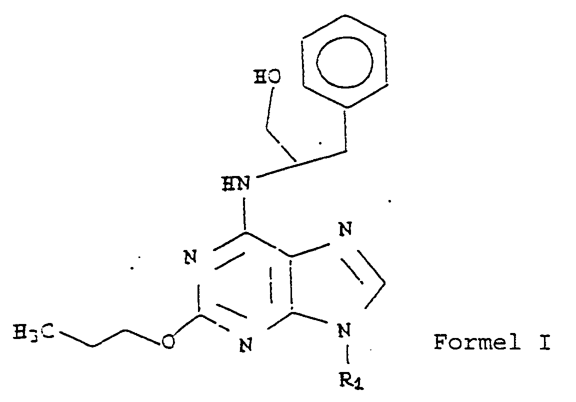 wobei R₁ ein Wasserstoffatom oder eine Phenylgruppe bedeutet.

2. Verbindung nach Anspruch 1, die β-[(2-Propoxy-1H-purin-6-yl)amino]benzolpropanol ist.

3. Verbindung der Formel, 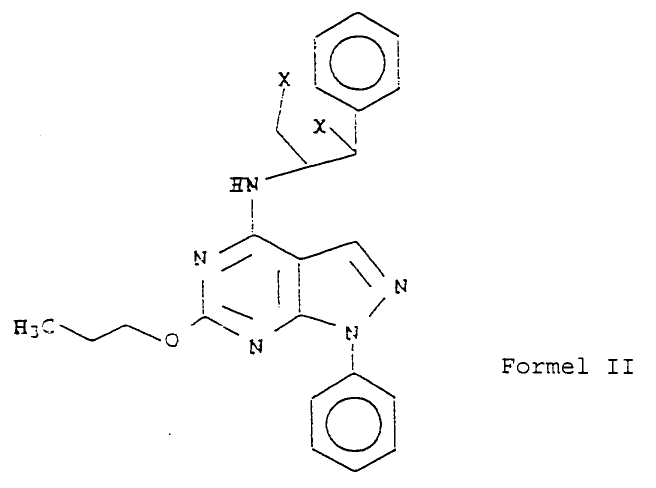 wobei die Reste X jeweils unabhängig voneinander ein Wasserstoffatom oder eine Hydroxylgruppe darstellen.

4. Verbindung nach Anspruch 3, die (R)-N-(1-Methyl-2-phenylethyl)-1-phenyl-6-propoxy-1H-pyrazolo[3,4-d]pyrimidin-4-amin, (S)-N-(1-Methyl-2-phenylethyl)-1-phenyl-6-propoxy-1H-pyrazolo[3,4-d]pyrimidin-4-amin, [S-(R*,S*)]-α-[1-[(1-Phenyl-6-propoxy-1H-pyrazolo[3,4-d]pyrimidin-4-yl)amino]ethyl]benzolethanol, [R-(S*,R*)]-α-[1-[(1-Phenyl-6-propoxy-1H-pyrazolo[3,4-d]pyrimidin-4-yl)aminolethyl]benzolethanol, (S)-β-[(1-Phenyl-6-propoxy-1H-pyrazolo[3,4-d]pyrimidin-4-yl)amino]benzolpropanol oder (R)-β-[(1-Phenyl-6-propoxy-1H-pyrazolo[3,4-d]pyrimidin-4-yl)amino]benzolpropanol ist.

5. Verbindung nach einem der Ansprüche 1 bis 4 zur Verwendung als therapeutisches Mittel.

6. Arzneimittel, das eine Verbindung gemäß einem der Ansprüche 1 bis 4 und einen physiologisch verträglichen Träger umfaßt.

7. Verfahren zur Herstellung eines Arzneimittels, umfassend die Vereinigung einer Verbindung, die in den Ansprüchen 1 bis 4 definiert ist, mit einem pharmazeutisch verträglichen Träger.

8. Verfahren zur Herstellung einer Verbindung, die in Anspruch 1 oder Anspruch 2 definiert ist, umfassend die Umsetzung einer Verbindung der Formel IV, 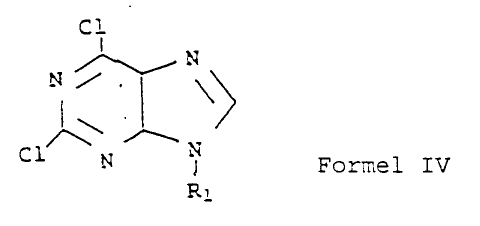 wobei R₁ ein Wasserstoffatom oder eine Phenylgruppe bedeutet, mit Propanol, indem die Verbindung der Formel IV mit Propanol in Ethanol in Gegenwart von Triethylamin für einen Zeitraum von 1 bis 20 Stunden bei einer Temperatur von 25 bis 140°C in Kontakt gebracht wird, wodurch das Zwischenprodukt der Formel V erhalten wird, 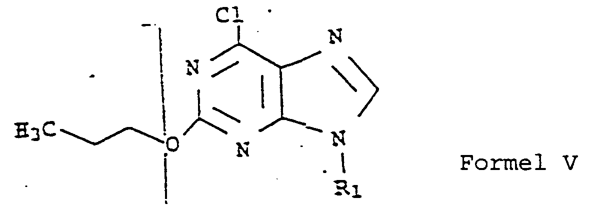 und anschließend eine Verbindung der Formel V mit einer Verbindung der Formel III für einen Zeitraum von 1 bis 20 Stunden bei einer Temperatur von 25 bis 140°C umgesetzt wird, wodurch das gewünschte Produkt der Formel I erhalten wird.

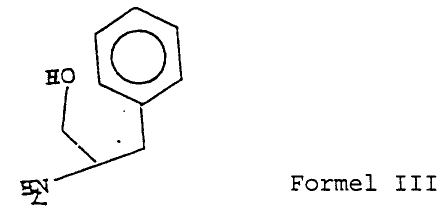

9. Verfahren zur Herstellung einer Verbindung gemäß Anspruch 3 oder Anspruch 4, umfassend die Umsetzung einer Verbindung der Formel IV' 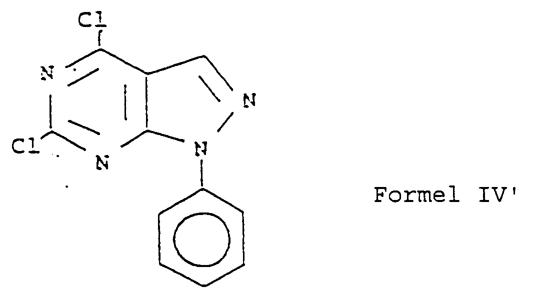 mit Propanol, indem die Verbindung der Formel IV' mit Propanol in Ethanol in Gegenwart von Triethylamin für einen Zeitraum von 1 bis 20 Stunden bei einer Temperatur von 25 bis 140°C in Kontakt gebracht wird, wodurch das Zwischenprodukt der Formel V' erhalten wird, 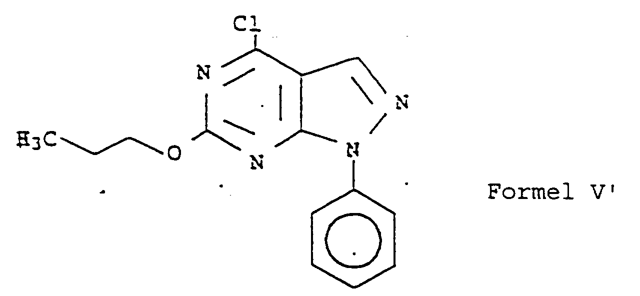 und anschließend eine Verbindung der Formel V' mit einer Verbindung der Formel III' für einen Zeitraum von 1 bis 20 Stunden bei einer Temperatur von 25 bis 140°C umgesetzt wird, wodurch das gewünschte Produkt der Formel II erhalten wird, 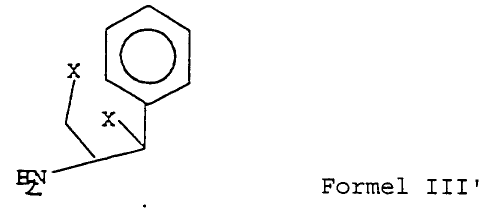 wobei die Reste X jeweils unabhängig voneinander ein Wasserstoffatom oder eine Hydroxylgruppe bedeuten.

## Revendications

1. Composé de formule : 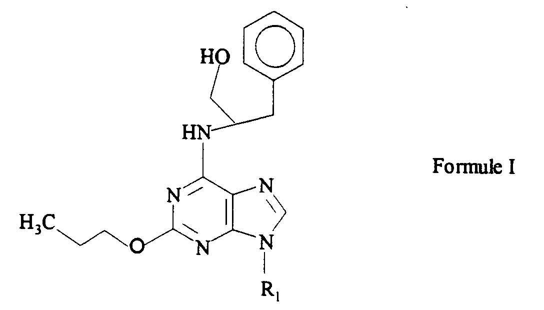 où R₁ est hydrogène ou phényle.

2. Composé selon la revendication 1 qui est le β-[(2-propoxy-1H-purin-6-yl)amino]benzènepropanol.

3. Composé de formule : 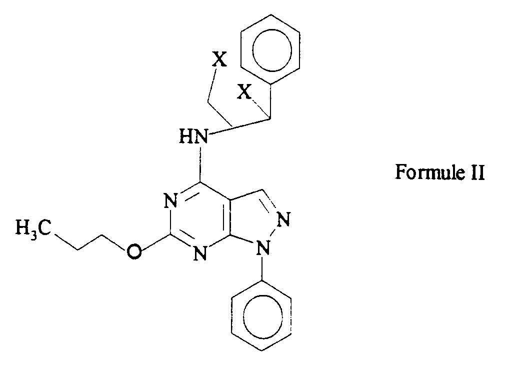 où chaque X est indépendamment hydrogène ou hydroxyle.

4. Composé selon la revendication 3 qui est la (R)-N-(1-méthyl-2-phényléthyl)-1-phényl-6-propoxy-1H-pyrazolo[3,4-d]pyrimidin-4-amine, la (S)-N-(1-méthyl-2-phényléthyl)-1-phényl-6-propoxy-1H-pyrazolo[3,4-d]pyrimidin-4-amine, le [S-(R*,S*)]-α-(1-[(1-phényl-6-propoxy-1H-pyrazolo[3,4-d]pyrimidin-4-yl)amino]éthyl]benzèneéthanol, le [R-(S*,R*)]-α-(1-[(1-phényl-6-propoxy-1H-pyrazolo[3,4-d]pyrimidin-4-yl)amino]éthyl]benzèneéthanol, le (S)-β-[(1-phényl-6-propoxy-1H-pyrazolo[3,4-d]pyrimidin-4-yl)amino]benzènepropanol ou le (R)-β-[(1-phényl-6-propoxy-1H-pyrazolo[3,4-d]pyrimidin-4-yl)amino]benzènepropanol.

5. Composé selon l'une quelconque des revendications 1 à 4, destiné à être utilisé comme agent thérapeutique.

6. Composition pharmaceutique comprenant un composé selon l'une quelconque des revendications 1 à 4 et un support physiologiquement acceptable.

7. Procédé de préparation d'une composition pharmaceutique comprenant la combinaison d'un composé tel que défini dans les revendications 1 à 4 avec un support pharmaceutiquement acceptable.

8. Procédé de préparation d'un composé tel que défini dans la revendication 1 ou 2, comprenant :
la réaction d'un composé selon la formule IV : 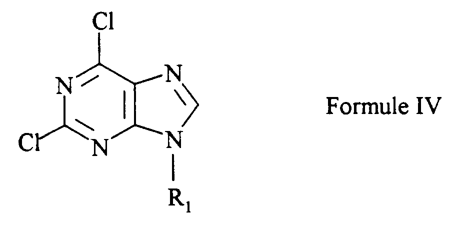 où R₁ est hydrogène ou phényle
avec le propanol par mise en contact du composé selon la formule IV avec du propanol dans l'éthanol en présence de triéthylamine pendant une durée de 1 à 20 h à une température de 25°C à 140°C pour obtenir à partir de ceux-ci le produit intermédiaire selon la formule V 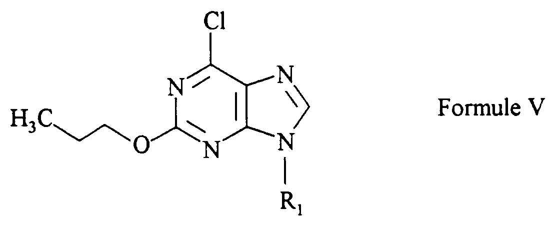 puis la réaction d'un composé selon la formule V avec un composé selon la formule III pendant une durée de 1 à 20 h à une température de 25 à 140°C pour obtenir à partir de ceux-ci le produit voulu selon la formule I

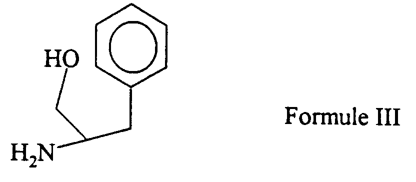

9. Procédé de préparation d'un composé selon la revendication 3 ou 4 comprenant la réaction d'un composé selon la formule IV' 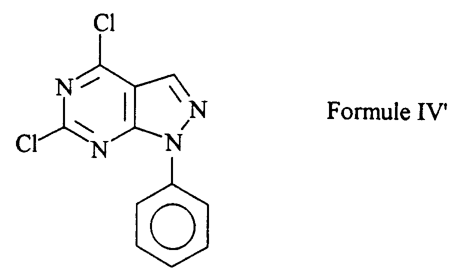 avec le propanol par mise en contact du composé selon la formule IV' avec du propanol dans l'éthanol en présence de triéthylamine pendant une durée de 1 à 20 h à une température de 25 à 140°C pour obtenir à partir de ceux-ci le produit intermédiaire selon la formule V' 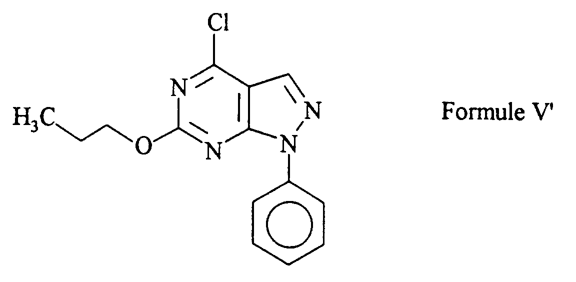 puis la réaction d'un composé selon la formule V' avec un composé selon la formule III' pendant une durée de 1 à 20 h à une température de 25 à 140°C pour obtenir, à partir de ceux-ci, le produit voulu selon la formule II 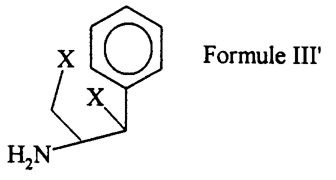 où chaque X est indépendamment hydrogène ou hydroxyle.
